(19) Europäisches Patentamt
European Patent Office
Office européen des brevets

(11) EP 0 617 027 B1

(12) FASCICULE DE BREVET EUROPEEN

(45) Date de publication et mention de la délivrance du brevet: **07.06.2000 Bulletin 2000/23**

(51) Int. Cl.[7]: **C07D 295/08**, A61K 31/495

(21) Numéro de dépôt: **94400609.7**

(22) Date de dépôt: **22.03.1994**

# (54) Nouveaux composés de N-benzylpipérazine leur procédé de préparation et les compositions pharmaceutiques les renfermant

N-Benzylpiperazinverbindungen, Verfahren zu ihrer Herstellung und sie enthaltende Zusammensetzungen

N-Benzylpiperazinderivatives, process for their preparation and pharmaceutical compositions containing them

(84) Etats contractants désignés:
**AT BE CH DE DK ES FR GB GR IE IT LI LU NL PT SE**

(30) Priorité: **24.03.1993 FR 9303364**

(43) Date de publication de la demande:
**28.09.1994 Bulletin 1994/39**

(73) Titulaire: **ADIR ET COMPAGNIE**
**92415 Courbevoie Cédex (FR)**

(72) Inventeurs:
- **Wierzbicki, Michel**
  **F-78620 l Etang La Ville (FR)**
- **Lepagnol, Jean**
  **F-28210 Chaudon (FR)**
- **Tillement, Jean-Paul**
  **F-77590 Bois le Roi (FR)**
- **Testa, Bernard**
  **CH-1005 Lausanne (CH)**
- **Rolland, Yves**
  **F-92170 Vanves (FR)**

(74) Mandataire: **Reverbori, Marcelle**
**ADIR**
**1, rue Carle Hébert**
**92415 Courbevoie Cédex (FR)**

(56) Documents cités:

**EP-A- 0 326 379**
**EP-A- 0 453 365**
**EP-A- 0 533 579**
**DE-A- 1 445 138**
**FR-A- 1 302 958**
**FR-A- 2 493 316**
**FR-M- 805**

- **CHEMICAL ABSTRACTS, vol. 79, no. 5, 6 Aoüt 1973, Columbus, Ohio, US; abstract no. 32098k, page 462 ;colonne G ; & JP-A-7 332 889 (NISSHIN FLOUR MILLING CO)**
- **CHEMICAL & PHARMACEUTICAL BULLETIN vol. 35, no. 7 , Juillet 1987 , TOKYO pages 2774 - 2781 HIROSHI OHTAKA ET. AL.**
- **CHEMICAL & PHARMACEUTICAL BULLETIN vol. 37, no. 11 , Novembre 1989 , TOKYO pages 3122 - 3124 HIROSHI OHTAKA ET. AL.**
- **J. PHARMACOBIO-DYN. vol. 14, no. 8 , Aoüt 1991 pages 449 - 459 KAWASHIMA, TSUNEO ET. AL.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

La présente invention a pour objet les nouveaux composés de N-benzyl pipérazine, leur procédé de préparation et les compositions pharmaceutiques les renfermant.

Elle concerne particulièrement les composés de formule I :

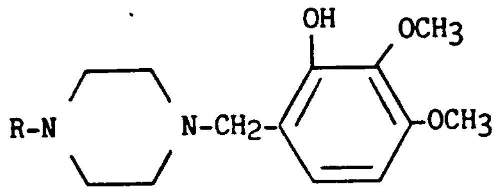

(I)

dans laquelle R représente :

1) un atome d'hydrogène, ou
2) un radical alkyle renfermant de 1 à 20 atomes de carbone, en chaîne droite ou ramifiée, éventuellement mono- ou poly- substitué par :

a- un radical cycloalkyle, renfermant de 3 à 7 atomes de carbone, éventuellement substitué par un radical phényle lui-même éventuellement substitué par un ou plusieurs atomes d'halogène ou radicaux alkyle ou alkoxy ayant chacun de 1 à 5 atomes de carbone,
b- un radical phényle lui-même éventuellement substitué par un ou plusieurs atomes d'halogène ou radicaux alkyle ou alkoxy ayant chacun de 1 à 5 atomes de carbone, ou
c- un radical $OR_1$ dans lequel $R_1$ représente :

- un atome d'hydrogène, ou
- un radical alkyle contenant de 1 à 5 atomes de carbone en chaîne droite ou ramifiée,

à la condition que R soit différent du groupement di(4-fluorophényl)méthyle.

L'état antérieur de la technique est illustré notamment par :

- les brevets français 1 302 958 et 805 M qui concernent respectivement la préparation de N-trialkoxy benzyl pipérazines et l'utilisation comme médicament à action vaso-dilatatrice de la 2,3,4-triméthoxybenzyl pipérazine,
- les articles d'Hiroshi Ohtaka et coll., Chem. Pharm. Bull. 35, 2774-3275 (1987) et Chem. Pharm. Bull. 37, 11, 3122-3124 (1989) qui mentionnent des dérivés de trimétazidine ayant une activité vasodilatatrice, et la synthèse de 1-[bis(4-fluorophényl)méthyl]4-(2-hydroxy 3,4-diméthoxybenzyl) pipérazine, et
- l'article de Tsunéo Kawashima et coll., J. Pharmacobio-Dyn, 14, 449-459 (1991) relatif à l'isolation et l'identification de nouveaux métabolites du KB-2796 dont entre autres la 1-[bis(4-fluorophényl)méthyl]4-(2-hydroxy 3,4-diméthoxybenzyl)pipérazine.

Les dérivés de la présente invention outre le fait qu'ils aient une structure chimique nouvelle par rapport à celle des dérivés les plus proches de l'art antérieur, présentent une activité pharmacologique et des propriétés thérapeutiques intéressantes.

Ils s'opposent notamment aux conséquences des déséquilibres du métabolisme oxydatif cérébral et tout particulièrement aux conséquences des phénomènes d'hyperoxydation et de peroxydation (stress oxydatif).

Ils peuvent de ce fait être utilisés dans le traitement des syndromes ischémiques aigus, transitoires ou progressifs, et des maladies nerveuses liées au vieillissement normal ou pathologique tel que la maladie d'Alzheimer.

La présente invention a également pour objet le procédé de préparation des composés de formule I caractérisé en ce que :

1) Le composé de formule I dans laquelle R représente un atome d'hydrogène est préparé par débenzylation du composé N-benzylé correspondant.
Il est avantageux d'effectuer cette débenzylation au moyen d'hydrogène en présence d'un catalyseur tel que par exemple le palladium.
2) Les composés de formule I dans laquelle R prend les significations autre qu'hydrogène, énoncées précédemment, sont préparés par action d'un réducteur sur les composés de formule II :

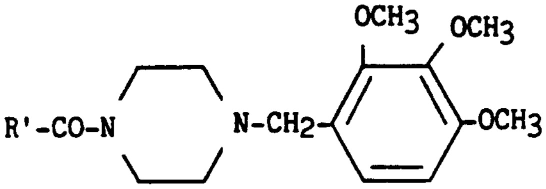

(II)

dans laquelle R' représente :

a- un atome d'hydrogène ;

b- un radical phényle éventuellement substitué par un ou plusieurs atomes d'halogène ou un ou plusieurs radicaux alkyle ou alkoxy ayant chacun de 1 à 5 atomes de carbone ;

c- un radical cycloalkyle renfermant de 3 à 7 atomes de carbone éventuellement substitué par un radical phényle lui-même éventuellement substitué par un ou plusieurs atomes d'halogène ou un ou plusieurs radicaux alkyle ou alkoxy ayant chacun de 1 à 5 atomes de carbone ; ou

d- un radical alkyle contenant de 1 à 19 atomes de carbone, en chaîne droite ou ramifiée, éventuellement mono- ou poly-substitué par :

α- un radical cycloalkyle renfermant de 3 à 7 atomes de carbone, éventuellement subsituté par un radical phényle, lui-même éventuellement substitué par un ou plusieurs atomes d'halogène ou un ou plusieurs radicaux alkyle ou alkoxy ayant chacun de 1 à 5 atomes de carbone ;

β- un radical phényle, lui-même éventuellement subsituté par un ou plusieurs atomes d'halogène ou un ou plusieurs radicaux alkyle ou alkoxy ayant chacun de 1 à 5 atomes de carbone ; ou

γ- un radical choisi parmi les groupes de formule : $-O-COR_3$, $-COOR_3$ ou

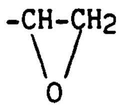

(dans lesquelles $R_3$ représente un atome d'hydrogène ou un radical alkyle contenant de 1 à 5 atomes de carbone), selon que l'on veut préparer un composé de formule I dans laquelle le substituant R contient respectivement un groupe :

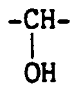 ,

$-CH_2-OH$ ou

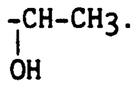

On réalise ainsi simultanément sur le composé II la réduction de la fonction CO liée au noyau pipérazine et la déméthylation quasi exclusive du groupe méthoxy situé en position 2 du groupement triméthoxybenzyle.

Le composé I ainsi obtenu peut être purifié selon les méthodes physiques et chimiques classiques.

Les matières premières de formule II ont été préparées par acylation de la trimétazidine.

Les composés de formule I peuvent être transformés en sels d'addition avec les acides, sels qui font, à ce

titre, partie de la présente invention. Comme acides utilisables pour la formation de ces sels, on peut citer, par exemple, dans la série inorganique, les acides chlorhydrique, bromhydrique, sulfurique, nitrique, phosphorique et dans la série organique, les acides acétique, propionique, maléique, fumarique, tartrique, oxalique, benzoïque, méthanesulfonique et iséthionique.

Les composés de formule I sont des huiles ou des composés cristallisés à bas point de fusion. Il est donc utile d'en synthétiser les sels (généralement mono- ou di-chlorhydrates) pour obtenir des produits cristallisés solubles dans l'eau.

Les composés de formule I et leurs sels d'addition physiologiquement tolérables possèdent des propriétés pharmacologiques et thérapeutiques intéressantes.

En effet, il est bien connu que lors du vieillissement cérébral physiologique ou pathologique (démences) et au cours des accidents cérébraux, les neurones sont particulièrement vulnérables vis-à-vis des déséquilibres du métabolisme oxydatif et tout particulièrement vis-à-vis des phénomènes d'hyperoxydation réactionnelle ou stress oxydatif. C'est pourquoi la formation des radicaux libres oxygénés est considérée comme l'une des causes de la mort neuronale aigüe ou progressive.

Cette vulnérabilité particulière des neurones aux processus de peroxydation peut s'expliquer en partie par les fortes concentrations de fer et d'ascorbate présentes dans le cerveau et par les faibles taux cérébraux d'enzymes antioxydants.

Les phénomènes pathologiques liés à l'hyperoxydation touchent tout autant les structures lipidiques cellulaires que les protéines membranaires ou cytosoliques. Ils conduisent à la désorganisation structurale, ce qui a été démontré tant lors du vieillissement cérébral que lors d'ischémie aigüe.

Il est possible de montrer expérimentalement les effets délétères de l'hyperoxygénation-hyperoxydation tant in vitro qu'in vivo.

C'est ainsi que les dérivés de la présente invention ont été rationnellement et préférentiellement étudiés in vivo dans un modèle de convulsions induites par oxygénation hyperbare et dans un modèle de commotion cérébrale chez la Souris. Ces deux modèles ont été choisis pour leur capacité à mettre en jeu des phénomènes d'hypermétabolisme cérébral, donc de stress oxydatif, dont les conséquences comportementales peuvent être atténuées par les composés de référence dits antiradicalaires ("free radicals scavengers") ou inversement, aggravées par les agents facilitant les phénomènes de peroxydation.

Dans ces conditions, les composés de la présente invention ont montré des effets protecteurs importants vis-à-vis des aggressions cérébrales expérimentales. Ces effets ont été obtenus à doses inférieures et avec une biodisponibilité cérébrale bien supérieure à celles des composés de l'art antérieur pris en référence.

En s'opposant nettement aux phénomènes délétères liés au détournement de l'utilisation physiologique de l'oxygène, les composés de la présente invention peuvent être utilisés en thérapeutique pour le traitement des maladies neuronales dûes au dysfonctionnement du métabolisme oxydatif et notamment dûes au stress oxydatif cellulaire. Ces pathologies neuronales qui peuvent être d'origine centrale, périphérique ou médullaire, entraînent la mort neuronale aigüe ou progressive. Ce sont par exemple les ischémies constituées ou transitoires, les traumatismes, le vieillissement cérébral et les maladies neurodégénératives telles que, par exemple, les maladies d'Alzheimer, de Parkinson, de Pick et de Huntington.

La présente invention a également pour objet les compositions pharmaceutiques contenant comme principe actif un composé de formule I ou un de ses sels physiologiquement tolérable mélangé ou associé à un excipient pharmaceutique approprié.

Les compositions pharmaceutiques ainsi obtenues se présentent généralement sous forme dosée et peuvent contenir de 0,1 à 300 mg de principe actif.

Elles peuvent revêtir la forme de comprimés, dragées, gélules, suppositoires, solutions injectables ou buvables et être administrées par voie orale, rectale, intraveineuse ou parentérale.

La posologie varie selon l'âge et le poids du patient, la voie d'administration, la nature de la maladie et les traitements associés. Elle s'échelonne généralement de 0,1 à 300 mg de principe actif de 1 à 3 fois par jour.

Les exemples suivants illustrent la présente invention.

**A/ PREPARATION DES MATIERES PREMIERES DE FORMULE II** par acylation de la trimétazidine.

Préparation de N-benzoyl trimétazidine

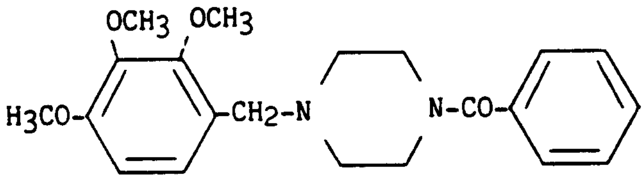

On additionne simultanément dans un réacteur 53,2 g (0,2 mole) de trimétazidine dans 400 ml d'éther et 14 g (0,1 mole) de chlorure de benzoyle dans 400 ml d'éther, en agitant vigoureusement à température ambiante. Lorsque l'addition est terminée, l'agitation est encore maintenue pendant 3 heures, puis le mélange est filtré. Le résidu est lavé par trois fois 200 ml d'éther. La phase éthérée est séchée et le solvant distillé. Une première fraction de 29 g est ainsi obtenue. Le résidu de filtration est dissout dans 300 ml d'eau. La phase aqueuse est extraite par deux fois 200 ml d'éther. On récupère ainsi une deuxième fraction de 3,7 g que l'on ajoute à la première, ce qui donne ainsi 32,7 g de N-benzoyltrimétazidine (Rendement : 88 %).

En opérant selon le même procédé, ont été obtenues les :

N-formyl trimétazidine,
N-acétyl trimétazidine,
N-propionyl trimétazidine,
N-isobutyryl trimétazidine,
N-butyryl trimétazidine,
N-hexylcarbonyl trimétazidine,
N-pentadécylcarbonyl trimétazidine,
N-heptadécylcarbonyl trimétazidine,
N-(3-éthoxycarbonyl propionyl) trimétazidine,
N-(4-éthoxycarbonyl 3,3-diméthyl butyryl) trimétazine
N-phénylcyclopropylcarbonyl trimétazidine,
N-(7-éthoxycarbonyl heptanoyl)trimétazidine.

**B/ PREPARATION DES COMPOSES DE FORMULE I**

Exemple 1

N-éthyl N'-(2-hydroxy 3,4-diméthoxybenzyl) pipérazine :

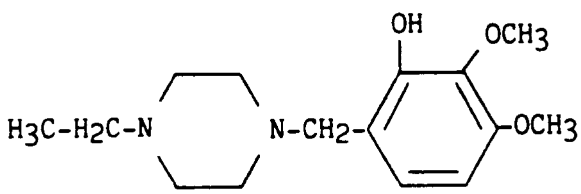

30,8 g (0,1 mole) de N-acétyl trimétazidine, 600 ml d'éther et 11,1 g (0,3 mole) de Li Al $H_4$ sont amenés puis maintenus au reflux pendant 24 heures sous agitation vigoureuse, puis on laisse le mélange revenir à température ambiante et maintient l'agitation pendant encore 12 heures. Après quoi, on hydrolyse le mélange successivement par 10 ml d'eau puis 10 ml de soude 4N et enfin 30 ml d'eau. Le mélange est filtré et le résidu lavé par trois fois 200 ml d'éther. Les phases éthérées sont écartées et le résidu est alors repris par trois fois 300 ml de $CH_2Cl_2$. La phase organique obtenue après filtration est séchée sur sulfate de magnésium, puis le solvant est éliminé par évaporation sous vide. Le résidu

(environ 20 g) est filtré sur 150 g de silice en éluant avec un mélange de chloroforme et d'éthanol (97,5 % - 2,5 %). On recueille ainsi directement 18 g de N-éthyl N'-(2-hydroxy 3,4-diméthoxybenzyl)pipérazine (Rendement : 64 %).

0,01 mole du composé ainsi obtenu dissout dans le minimum d'éther anhydre est additionnée à une quantité deux fois stoechiométrique d'HCl en solution dans l'éther. Un précipité abondant se forme aussitôt. On le recueille par filtration et le recristallise dans un mélange éthanoléther. On obtient ainsi le dichlorhydrate de N-éthyl N'-(2-hydroxy 3,4-diméthoxybenzyl)pipérazine, PF > 250 °C.

Exemples 2 à 13 :

En opérant selon la méthode décrite dans l'exemple 1, ont été préparés les composés ci-après exemplifiés :

2) N-méthyl N'-(2-hydroxy 3,4-diméthoxybenzyl)pipérazine et son dichlorhydrate PF > 230 °C avec décomposition (éther).
3) N-propyl N'-(2-hydroxy 3,4-diméthoxybenzyl)pipérazine et son dichlorhydrate PF : 230-232 °C.
4) N-butyl N′-(2-hydroxy 3,4-diméthoxybenzyl)pipérazine et son dichlorhydrate PF : 240 °C avec sublimation.
5) N-isobutyl N'-(2-hydroxy 3,4-diméthoxybenzyl)pipérazine et son dichlorhydrate PF : 225 °C.
6) N-heptyl N'-(2-hydroxy 3,4-diméthoxybenzyl)pipérazine et son dichlorhydrate PF : 217 °C.
7) N-hexadécyl N'-(2-hydroxy 3,4-diméthoxybenzyl)pipérazine et son dichlorhydrate PF : 206-210 °C (isopropanol).
8) N-octadécyl N'-(2-hydroxy 3,4-diméthoxybenzyl)pipérazine et son dichlorhydrate PF : 221 °C (ether).
9) N-(4-hydroxybutyl) N′-(2-hydroxy 3,4-diméthoxybenzyl)pipérazine et son dichlorhydrate PF > 228 °C avec sublimation (éther).
10) N-(7-hydroxyoctyl) N′-(2-hydroxy 3,4-diméthoxybenzyl)pipérazine et son dichlorhydrate PF > 210 °C avec décomposition.
11) N-(5-hydroxy 3,3-diméthylpentyl) N′-(2-hydroxy 3,4-diméthoxybenzyl) pipérazine et son dichlorhydrate PF > 240 °C avec sublimation.
12) N-benzyl N'-(2-hydroxy 3,4-diméthoxybenzyl)pipérazine et son dichlorhydrate PF > 240 °C avec sublimation (éther).
13) E N-[(2-phénylcyclopropyl)méthyl] N′-(2-hydroxy 3,4-diméthoxybenzyl) pipérazine et son dichlorhydrate PF : 212 °C (isopropanol/eau).

Exemple 14

N-(2-hydroxy 3,4-diméthoxybenzyl)pipérazine :

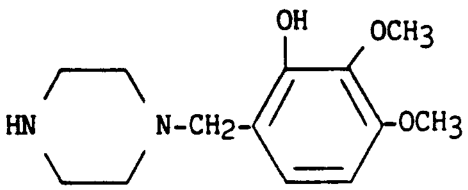

6,8 g (0,02 mole) de N-benzyl N′-(2-hydroxy 3,4-diméthoxybenzyl)pipérazine, sont dissouts dans 200 ml d'éthanol à 60 °C. On y ajoute 1,1 g de charbon palladié à 5 % et hydrogène l'ensemble sous une pression de 5 500 hPa. Après quoi, la solution alcoolique est filtrée et le solvant distillé. L'huile obtenue est chromatographiée sur 150 g de silice. Une première élution au dichlorométhane élimine les impuretés peu polaires du mélange. La silice est ensuite lavée avec un mélange $CH_2Cl_2/CH_3OH$ (90/10). On obtient ainsi 3,78 g de N-(2-hydroxy 3,4-diméthoxybenzyl)pipérazine (Rendement 60 %) que l'on transforme ensuite en son dichlorhydrate (PF > 190 °C) par addition de deux fois la quantité stoechiométrique d'HCl.

C/ **ETUDE PHARMACOLOGIQUE**

Exemple 15

a) **Principe**

Les déséquilibres du métabolisme oxydatif et plus particulièrement le stress oxydatif sont considérés

comme l'une des causes majeures de la mort neuronale aigüe ou progressive. En effet, le détournement de l'utilisation physiologique de l'oxygène vers la formation de radicaux libres oxygénés entraîne une désorganisation de la structure cellulaire, tant lipidique que protéique.

Il est désormais bien démontré que ces phénomènes de peroxydation sont caractéristiques du vieillissement cérébral normal ou de type démentiel et des accidents vasculaires cérébraux.

L'oxygénation hyperbare in vivo permet de reproduire expérimentalement et de façon accélérée le stress oxydatif qui se manifeste chez la Souris par des crises convulsives pouvant conduire à la mort. Dans ces conditions, les agents protecteurs antioxydants (glutathion, scavengers) retardent l'apparition de la crise convulsive tandis que les agents prooxydants ($H_2O_2$, ascorbate, $Fe^{++}$) la facilitent.

De la même manière, un choc crânien non traumatisant entraîne un hypermétabolisme réactionnel qui se manifeste par une crise convulsive. Celle-ci conduit à une phase de coma transitoire dont les agents protecteurs antioxydants (scavengers) peuvent diminuer la durée.

Les composés de l'invention ont été étudiés dans ces deux épreuves (oxygénation hyperbare, commotion cérébrale) selon des voies d'administration centrale (ICV : intracérébroventriculaire) ou périphérique (IV : intraveineuse ; IP : intrapéritonéale) afin d'étudier, outre l'intensité de leurs effets pharmacologiques, leur degré de biodisponibilité intracérébrale.

b) **Méthodologie**

. Oxygénation hyperbare chez la Souris.

Des Souris CD1 mâles (Charles-River) sont placées individuellement dans des caissons étanches où circule un courant d'oxygène pur à la pression de 6 atmosphères. Le temps d'apparition de la crise tonico-clonique généralisée est noté pour chaque animal. Dans ces conditions, la crise survient en 17 à 18 minutes chez les animaux témoins. Pour chaque composé étudié, le-temps moyen d'apparition de la crise est comparé à celui d'un lot témoin ne recevant que le solvant. Pour chaque composé, on détermine ainsi une dose efficace 50 (DE50)allongeant de 50 % le temps d'apparition de la crise. Les composés sont étudiés soit en administration ICV dans 5 µl (doses en mg/animal), soit en administration IV dans 5 ml/kg (doses en mg/kg), 30 minutes avant le test.

. Commotion chez la Souris.

Des Souris CD1 mâles (Charles-River) vigiles sont immobilisées au niveau du crâne par une tige métallique verticale terminée par un embout de teflon. Un poids coulissant autour de la tige est lâché d'une hauteur de 12 cm et le choc de ce poids avec l'embout de teflon se répercute sur la voûte crânienne de l'animal. Ce choc entraîne une crise convulsive puis le coma et parfois la mort des animaux. Le temps de reprise de locomotion spontanée est noté. Chez les animaux témoins, ce temps est d'environ 250 secondes.

Pour chaque dose de composé étudiée, un index de protection est calculé comme le temps médian de coma des animaux.

Pour chaque composé étudié, on détermine la dose efficace 66 (DE66) qui diminue le temps médian de coma de 66 %.

Les composés sont étudiés en administration IP sous un volume de 20 ml/kg (dose en mg/kg) 30 minutes avant le test.

c) **Résultats**

. Oxygénation hyperbare chez la Souris

Dans nos conditions drastiques de stress oxydatif, les composés de la présente invention exercent des effets protecteurs beaucoup plus intéressants que ceux des composés pris en référence, soit dans l'art antérieur, la trimétazidine, soit parmi les composés antiradicalaires les plus puissants, U78517F (lazaroïde).

En effet, bien qu'ils exercent des effets comparables par voie ICV, leurs effets protecteurs par voir IV, voie d'administration thérapeutique, sont bien supérieurs et le rapport des doses efficaces IV/IVC montre qu'ils possèdent une bien meilleure biodisponibilité cérébrale. De plus, ces effets s'exercent selon une relation dose-effet linéaire sur une large gamme de doses, ce qui n'est pas le cas notamment de la trimétazidine pour laquelle la gamme de doses actives est très étroite et biphasique.

Le tableau suivant regroupe ces résultats :

| | ED50 IV | $\frac{ED50\ IV}{ED50\ ICV}$ | Relation dose-effet |
|---|---|---|---|
| Trimétazidine | 2 | 25 | Biphasique |
| U78517F | 5 | 16 | Linéaire |
| Exemple 10 | 0,5 | 1 | Linéaire |
| Exemple 13 | 0,2 | 1 | Linéaire |
| Exemple 14 | 0,6 | 6 | Linéaire |

Commotion cérébrale chez la Souris

Comme dans le test précédent, les composés de l'invention exercent des effets protecteurs à doses plus faibles que celles des composés de référence. Ces effets se manifestent aussi selon une relation dose-effet linéaire, ce qui n'est pas, là encore, le cas de la trimétazidine qui n'est active significativement qu'à une seule dose (3 mg/kg IP).

| | ED66 | Relation dose-effet | Gamme de doses actives |
|---|---|---|---|
| Trimétazidine | 1 | Biphasique | 1 et 3 |
| U78517F | 3 | Linéaire | 3 et 10 |
| Exemple 1 | 0,2 | Linéaire | 0,1 à > 3 |
| Exemple 10 | 0,2 | Linéaire | 0,1 à > 3 |
| Exemple 14 | 0,5 | Biphasique | 0,1 à 3 |

d) **Conclusion**

Les composés de l'invention se distinguent nettement des composés pris en référence par l'intensité de leurs effets pharmacologiques qui s'exercent grâce à un tropisme cérébral préférentiel et selon une relation dose-effet linéaire donc pharmacologique. Ces effets sont supérieurs à ceux des composés de l'art antérieur comme à ceux des composés connus pour être les plus actifs vis-à-vis des désordres cellulaires dûs à l'hyperoxydation et au stress oxydatif.

Les composés de l'invention constituent donc des agents thérapeutiques originaux pour éviter et limiter la mort neuronale aigüe ou progressive.

**Revendications**

**1.** Les composés de N-benzylpipérazine de formule I :

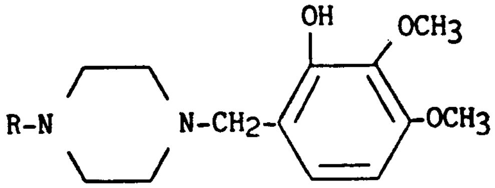

(I)

dans laquelle R représente :

1) un atome d'hydrogène, ou
2) un radical alkyle renfermant de 1 à 20 atomes de carbone, en chaîne droite ou ramifiée, éventuellement

mono- ou poly- substitué par :

a- un radical cycloalkyle, renfermant de 3 à 7 atomes de carbone, éventuellement substitué par un radical phényle lui-même éventuellement substitué par un ou plusieurs atomes d'halogène ou radicaux alkyle ou alkoxy ayant chacun de 1 à 5 atomes de carbone,
b- un radical phényle lui-même éventuellement substitué par un ou plusieurs atomes d'halogène ou radicaux alkyle ou alkoxy ayant chacun de 1 à 5 atomes de carbone, ou
c- un radical $OR_1$ dans lequel $R_1$ représente :

- un atome d'hydrogène, ou
- un radical alkyle contenant de 1 à 5 atomes de carbone en chaîne droite ou ramifiée ,

à la condition que R soit différent du groupement di(4-fluorophényl)méthyle.
ainsi que leurs sels d'addition physiologiquement tolérables avec des acides appropriés.

**2.** La N-éthyl N'-(2-hydroxy 3,4-diméthoxybenzyl)pipérazine et son dichlorhydrate.

**3.** La N-(7-hydroxyoctyl) N′-(2-hydroxy 3,4-diméthoxybenzyl)pipérazine et son dichlorhydrate.

**4.** La E N-[(2-phénylcyclopropyl)méthyl] N′-(2-hydroxy 3,4-diméthoxybenzyl)pipérazine et son dichlorhydrate.

**5.** La N-(2-hydroxy 3,4-diméthoxybenzyl)pipérazine et son dichlorhydrate.

**6.** Le procédé de préparation des composés de la revendication 1 caractérisé en ce que :

- dans le cas où R représente un atome d'hydrogène, on débenzyle la N-benzyl N′-(2-hydroxy 3,4-diméthoxybenzyl)pipérazine, au moyen d'hydrogène en présence d'un catalyseur ; et
- dans le cas où R prend les significations autre qu'hydrogène définies dans la revendication 1, on réduit les composés de formule II :

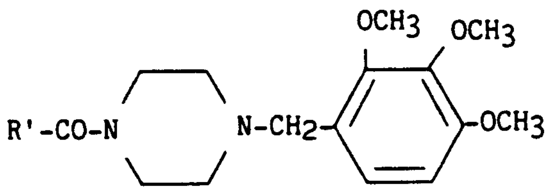

(II)

dans laquelle R′ représente :

a- un atome d'hydrogène ;
b- un radical phényle éventuellement substitué par un ou plusieurs atomes d'halogène ou un ou plusieurs radicaux alkyle ou alkoxy ayant chacun de 1 à 5 atomes de carbone ;
c- un radical cycloalkyle renfermant de 3 à 7 atomes de carbone éventuellement substitué par un radical phényle lui-même éventuellement substitué par un ou plusieurs atomes d'halogène ou un ou plusieurs radicaux alkyle ou alkoxy ayant chacun de 1 à 5 atomes de carbone ; ou
d- un radical alkyle contenant de 1 à 19 atomes de carbone, en chaîne droite ou ramifiée, éventuellement mono- ou poly-substitué par :

α- un radical cycloalkyle renfermant de 3 à 7 atomes de carbone, éventuellement substitué par un radical phényle, lui-même éventuellement substitué par un ou plusieurs atomes d'halogène ou un ou plusieurs radicaux alkyle ou alkoxy ayant chacun de 1 à 5 atomes de carbone ;
β- un radical phényle, lui-même éventuellement subsituté par un ou plusieurs atomes d'halogène ou un ou plusieurs radicaux alkyle ou alkoxy ayant chacun de 1 à 5 atomes de carbone ; ou
γ- un radical choisi parmi les groupes de formule : $-O-COR_3-$, $-COOR_3$ ou

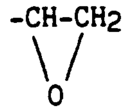

(dans lesquelles $R_3$ représente un atome d'hydrogène ou un radical alkyle contenant de 1 à 5 atomes de carbone), selon que l'on veut préparer un composé de formule I dans laquelle le substituant R contient respectivement un groupe :

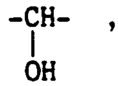

$-CH_2-OH$ ou

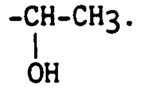

**7.** Les compositions pharmaceutiques contenant comme principe actif au moins un composé selon l'une des revendications 1 à 5, avec un ou plusieurs excipients pharmaceutiques appropriés.

**8.** Les compositions pharmaceutiques selon la revendication 7 présentées sous une forme convenant pour le traitement des maladies neuronales dues au dysfonctionnement du métabolisme oxydatif.

**Claims**

**1.** N-benzylpiperazine compounds of formula I :

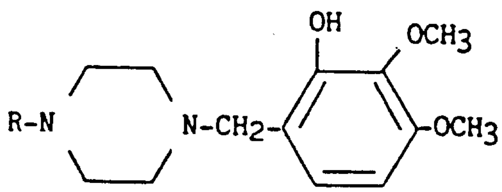

(I)

wherein R represents :

1) a hydrogen atom, or
2) a straight-chain or branched alkyl radical containing from 1 to 20 carbon atoms which is optionally mono- or poly-substituted by :

a- a cycloalkyl radical having from 3 to 7 carbon atoms optionally substituted by a phenyl radical which is itself optionally substituted by one or more halogen atoms or alkyl or alkoxy radicals each having from 1 to 5 carbon atoms,
b- a phenyl radical itself optionally substituted by one or more halogen atoms or alkyl or alkoxy radicals each having from 1 to 5 carbon atoms, or
c- an $OR_1$ radical in which $R_1$ represents :

- a hydrogen atom, or

- a straight-chain or branched alkyl radical containing from 1 to 5 carbon atoms;

with the proviso that R is other than the di(4-fluorophenyl)methyl group,
and also the physiologically tolerable addition salts thereof with appropriate acids.

**2.** N-ethyl-N'-(2-hydroxy-3,4-dimethoxybenzyl)-piperazine and its dihydrochloride.

**3.** N-(7-hydroxyoctyl)-N'-(2-hydroxy-3,4-dimethoxybenzyl)-piperazine and its dihydrochloride.

**4.** E-N-[(2-phenylcyclopropyl)methyl]-N'-(2-hydroxy-3,4-dimethoxybenzyl)-piperazine and its dihydrochloride.

**5.** N-(2-hydroxy-3,4-dimethoxybenzyl)-piperazine and its dihydrochloride.

**6.** A process for the preparation of compounds of claim 1, characterised in that :

- in the case where R represents a hydrogen atom, N-benzyl N'-(2-hydroxy-3,4-dimethoxybenzyl)-piperazine is debenzylated by means of hydrogen in the presence of a catalyst; and
- in the case where R has one of the meanings given in claim 1 other than hydrogen, a compound of formula II:

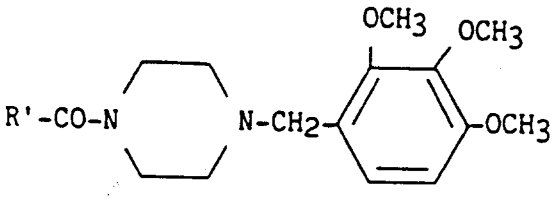

(II)

wherein R' represents :

a- a hydrogen atom;
b- a phenyl radical optionally substituted by one or more halogen atoms or by one or more alkyl or alkoxy radicals each having from 1 to 5 carbon atoms;
c- a cycloalkyl radical having from 3 to 7 carbon atoms optionally substituted by a phenyl radical which is itself optionally substituted by one or more halogen atoms or by one or more alkyl or alkoxy radicals each having from 1 to 5 carbon atoms; or
d- a straight-chain or branched alkyl radical containing from 1 to 19 carbon atoms which is optionally mono- or poly-substituted by :

α- a cycloalkyl radical having from 3 to 7 carbon atoms optionally substituted by a phenyl radical which is itself optionally substituted by one or more halogen atoms or by one or more alkyl or alkoxy radicals each having from 1 to 5 carbon atoms;
β- a phenyl radical, itself optionally substituted by one or more halogen atoms or by one or more alkyl or alkoxy radicals each having from 1 to 5 carbon atoms; or
τ- a radical selected from the groups of formulae : $-O-COR_3-$, $-COOR_3$ and

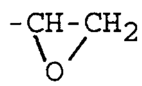

(wherein $R_3$ represents a hydrogen atom or an alkyl radical containing from 1 to 5 carbon atoms), according to whether it is desired to prepare a compound of formula I in which the substituent R contains, respectively, a

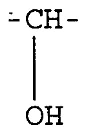 ,

$-CH_2-OH$ or

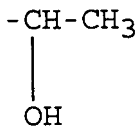

group,
is reduced.

**7.** Pharmaceutical compositions comprising as active ingredient at least one compound according to any one of claims 1 to 5, with one or more appropriate pharmaceutical excipients.

**8.** Pharmaceutical compositions according to claim 7 presented in a form suitable for the treatment of neuronal disorders resulting from the dysfunctioning of oxidative metabolism.

**Patentansprüche**

**1.** N-Benzylpiperazin-Verbindungen der Formel I:

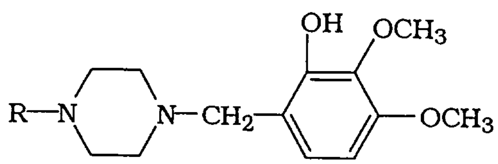

(I)

in der R:

1) ein Wasserstoffatom oder
2) eine Alkylgruppe mit 1 bis 20 Kohlenstoffatomen in gerader oder verzweigter Kette, die gegebenenfalls einfach oder mehrfach substituiert ist, durch:

a- eine Cycloalkylgruppe mit 3 bis 7 Kohlenstoffatomen, die gegebenenfalls durch eine Phenylgruppe substituiert ist, die ihrerseits gegebenenfalls durch ein oder mehrere Halogenatome oder Alkyl- oder Alkoxygruppen mit jeweils 1 bis 5 Kohlenstoffatomen substituiert ist,
b- eine Phenylgruppe, die ihrerseits gegebenenfalls durch ein oder mehrere Halogenatome oder Alkyl- oder Alkoxygruppen, die jeweils 1 bis 5 Kohlenstoffatome aufweisen, substituiert ist, oder
c- eine Gruppe $OR_1$, in der $R_1$:

- ein Wasserstoffatom oder
- eine Alkylgruppe mit 1 bis 5 Kohlenstoffatomen in gerader oder verzweigter Kette darstellt,

mit der Maßgabe bedeutet, daß R von der Di-(4-fluorphenyl)-methylgruppe verschieden ist,
sowie deren physiologisch verträgliche Additionssalze mit geeigneten Säuren.

**2.** N-Ethyl-N'-(2-hydroxy-3,4-dimethoxybenzyl)-piperazin und dessen Dihydrochlorid.

**3.** N-(7-Hydroxyoctyl)-N'-(2-hydroxy-3,4-dimethoxybenzyl)-piperazin und dessen Dihydrochlorid.

**4.** E-N-[(2-Phenylcyclopropyl)-methyl]-N'-(2-hydroxy-3,4-dimethoxybenzyl)-piperazin und dessen Dihydrochlorid.

**5.** N-(2-Hydroxy-3,4-dimethoxybenzyl)-piperazin und dessen Dihydrochlorid.

**6.** Verfahren zur Herstellung der Verbindungen nach Anspruch 1, dadurch gekennzeichnet, daß man:

- in dem Fall, da R ein Wasserstoffatom darstellt, N-Benzyl-N'-(2-hydroxy-3,4-dimethoxybenzyl)-piperazin mit Hilfe von Wasserstoff in Gegenwart eines Katalysators debenzyliert; und
- in dem Fall, da R eine von Wasserstoff verschiedene Bedeutung besitzt, wie sie in Anspruch 1 definiert ist, die Verbindungen der Formel II:

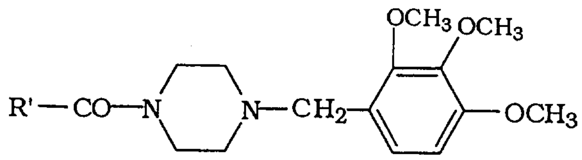

(II)

in der R':

a- ein Wasserstoffatom;
b- eine Phenylgruppe, die gegebenenfalls durch ein oder mehrere Halogenatome oder eine oder mehrere Alkyl- oder Alkoxygruppen mit jeweils 1 bis 5 Kohlenstoffatomen substituiert ist;
c- eine Cycloalkylgruppe mit 3 bis 7 Kohlenstoffatomen, die gegebenenfalls durch eine Phenylgruppe substituiert ist, die ihrerseits gegebenenfalls durch ein oder mehrere Halogenatome oder eine oder mehrere Alkyl- oder Alkoxygruppen mit jeweils 1 bis 5 Kohlenstoffatomen substituiert ist; oder
d- eine Alkylgruppe mit 1 bis 19 Kohlenstoffatomen in gerader oder verzweigter Kette, die gegebenenfalls einfach oder mehrfach substituiert ist durch:

$\alpha$- eine Cycloalkylgruppe mit 3 bis 7 Kohlenstoffatomen, die gegebenenfalls durch eine Phenylgruppe substituiert ist, die ihrerseits gegebenenfalls durch ein oder mehrere Halogenatome oder eine oder mehrere Alkyl- oder Alkoxygruppen mit jeweils 1 bis 5 Kohlenstoffatomen substituiert ist;
$\beta$- eine Phenylgruppe, die ihrerseits gegebenenfalls durch ein oder mehrere Halogenatome oder eine oder mehrere Alkyl- oder Alkoxygruppen mit jeweils 1 bis 5 Kohlenstoffatomen substituiert ist; oder
$\gamma$- eine Gruppe ausgewählt aus Gruppen der Formeln: $—O—COR_3$- , $—COOR_3$ oder

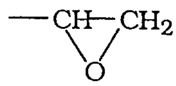

(in denen $R_3$ ein Wasserstoffatom oder eine Alkylgruppe mit 1 bis 5 Kohlenstoffatomen darstellt), in Abhängigkeit davon, ob man eine Verbindung der Formel I herstellen will, in der der Substituent R eine Gruppe:

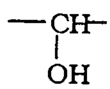

,

$—CH_2—OH$ bzw.

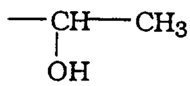

enthält, reduziert.

**7.** Pharmazeutische Zubereitungen enthaltend als Wirkstoff mindestens eine Verbindung nach einem der Ansprüche 1 bis 5 zusammen mit einem oder mehreren pharmazeutisch geeigneten Trägermaterialien.

**8.** Pharmazeutische Zubereitungen nach Anspruch 7 in einer Form, die zur Behandlung von Nervenerkrankungen geeignet sind, welche eine Folge sind einer Dysfunktion des oxidativen Stoffwechsels.